(19) Europäisches Patentamt
European Patent Office
Office européen des brevets

(11) **EP 2 621 428 B1**

(12) **EUROPÄISCHE PATENTSCHRIFT**

(45) Veröffentlichungstag und Bekanntmachung des Hinweises auf die Patenterteilung:
**22.10.2014 Patentblatt 2014/43**

(21) Anmeldenummer: **10763132.7**

(22) Anmeldetag: **30.09.2010**

(51) Int Cl.:
***A61F 9/008*** *(2006.01)*

(86) Internationale Anmeldenummer:
**PCT/EP2010/005977**

(87) Internationale Veröffentlichungsnummer:
**WO 2012/041352 (05.04.2012 Gazette 2012/14)**

(54) **EINRICHTUNG ZUR LASERTECHNISCHEN BEARBEITUNG DES HUMANEN AUGES**

DEVICE FOR LASERING THE HUMAN EYE

DISPOSITIF DE TRAITEMENT AU LASER DE L' OEIL HUMAIN

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(43) Veröffentlichungstag der Anmeldung:
**07.08.2013 Patentblatt 2013/32**

(73) Patentinhaber: **WaveLight GmbH**
**91058 Erlangen (DE)**

(72) Erfinder:
• **DONITZKY, Christof**
**90542 Eckental/Eschenau (DE)**

• **KLENKE, Joerg**
**90491 Nuernberg (DE)**
• **WUELLNER, Christian**
**91094 Bräuningshof (DE)**

(74) Vertreter: **Katérle, Axel**
**Wuesthoff & Wuesthoff**
**Patent- und Rechtsanwälte**
**Schweigerstraße 2**
**81541 München (DE)**

(56) Entgegenhaltungen:
**WO-A1-94/09849         DE-A1-102009 009 382**
**US-A1- 2008 319 428**

EP 2 621 428 B1

**Beschreibung**

Die Erfindung befasst sich mit der Erzeugung von Schnitten in der humanen Kornea mittels fokussierter und üblicherweise gepulster Laserstrahlung. Insbesondere befasst sich die Erfindung mit der Durchführung einer LASIK-Behandlung und mit der Präparation eines LASIK-Flaps mittels solcher Laserstrahlung.

Eine häufig eingesetzte Technik zur Behebung von Fehlsichtigkeiten des menschlichen Auges, wie beispielsweise Kurz- oder Weitsichtigkeit oder/und Astigmatismus, ist die sogenannte LASIK. LASIK steht für Laser in-situ Keratomileusis und bezeichnet eine Technik, bei der an der Oberfläche der Kornea zunächst ein Scheibchen (Lamelle) freigeschnitten wird, das zur Seite geklappt wird, um die darunter liegenden Gewebebereiche der Kornea freizulegen. Diese freigelegten Gewebebereiche werden sodann mittels fokussierter UV-Laserstrahlung ablatierend behandelt, d.h. es wird nach Maßgabe eines individuell für den Patienten ermittelten Ablationsprofils korneales Material abgetragen.

Das freigeschnittene Oberflächenscheibchen der Kornea wird in der Fachwelt regelmäßig als Flap bezeichnet; es ist nicht vollständig von der übrigen Kornea losgelöst, sondern ist in einem Scharnierbereich, der in der Fachwelt gemeinhin als Hinge bezeichnet wird, noch mit dem übrigen kornealen Gewebe verbunden. Dies ermöglicht ein einfaches Wegklappen des Flaps und vor allem ein einfaches Zurückklappen des Flaps nach der Ablation. Wegen des Materialabtrags stellt sich nach Zurückklappen des Flaps eine veränderte Form der Korneavorderfläche ein. Damit einhergehend ergibt sich ein anderes Brechungsverhalten der Kornea und folglich des Gesamtsystems Auge. Durch geeignete Festlegung des Ablationsprofils kann erreicht werden, dass die Fehlsichtigkeit zumindest deutlich abgeschwächt wird und bestenfalls sogar nahezu vollständig behoben wird.

Für die Präparation des Flaps sind im Stand der Technik verschiedene Vorgehensweisen bekannt. Eine Vorgehensweise nutzt ein mechanisches Mikrokeratom, d.h. einen mikrochirurgischen Hobel, der mit einer üblicherweise oszillierend angetriebenen Schneidklinge in die Kornea einschneidet. Eine andere Vorgehensweise, die im Rahmen der Erfindung näher betrachtet wird, nutzt fokussierte ultrakurzpulsige Laserstrahlung zur Präparation des Flaps. Üblicherweise wird hierbei Laserstrahlung mit Pulsdauern im Femtosekundenbereich, jedenfalls aber im niedrigen Pikosekundenbereich eingesetzt. Für die Anbringung kornealer Schnitte muss die hierfür verwendete Laserstrahlung eine Wellenlänge oberhalb ca. 300 nm besitzen, um eine Einkopplung der Strahlungsenergie in die Tiefe des kornealen Gewebes zu ermöglichen. LASIK-Behandlungen, bei denen der Flap mittels solcher ultrakurzpulsiger Laserstrahlung präpariert wird, werden oftmals als Fs-LASIK bezeichnet.

Für die Erzeugung von Schnitten mittels fokussierter Laserstrahlung in transparentem Material (transparent für die Laserstrahlung) wird als physikalischer Effekt der sogenannte laserinduzierte optische Durchbruch genutzt. Dieser führt zu einer Photodisruption des bestrahlten Gewebes im Bereich des Fokus. Durch Nebeneinandersetzen einer Vielzahl solcher Photodisruptionen können zwei- und dreidimensionale Schnittfiguren in der Kornea (und auch in anderen Gewebeteilen des Auges, die freilich hier nicht weiter betrachtet werden) realisiert werden. Die Strahlungsparameter der Laserstrahlung können so eingestellt sein, dass jeder einzelne Laserpuls zu einer Photodisruption führt. Es ist gleichermaßen vorstellbar, die Strahlungsparameter so einzustellen, dass eine Photodisruption nur nach Einstrahlung mehrerer (mindestens zwei) Laserpulse auf im wesentlichen dieselbe Stelle auftritt.

Besonders bei der Korrektur einer Myopie (Kurzsichtigkeit) durch eine LASIK-Behandlung stellt sich das Problem, dass nach der Ablation der Flap nicht mehr optimal in die Wundfläche (korneales Bett) passen kann. Denn zur Korrektur einer Myopie findet der stärkste Materialabtrag gemeinhin im Zentrum der ablativ bearbeiteten optischen Zone statt. Der Krümmungsradius der optischen Zone nimmt dadurch gegenüber dem Zustand vor der Ablation ab. Dies geht mit einer Verringerung der längs der Oberfläche gemessenen Bogenlänge der optischen Zone einher. Wird der Flap nun auf das korneale Bett zurückgeklappt, kann es sein, dass er sich nicht perfekt in das Bett einschmiegt, sondern Falten im Flap entstehen. Diese auch als Striae bezeichnete Erscheinung kann unangenehme Visusbeeinträchtigungen des Patienten hervorrufen. Zur Behebung der Komplikationen infolge von Striae des Flaps kann beispielsweise daran gedacht werden, den Flap nach dem Zurückklappen auf das Bett zu erwärmen und glattzustreichen. Dies stellt jedoch eine zusätzliche Belastung des Patienten durch einen weiteren Behandlungsschritt dar.

Aus DE 10 2009 009 382 A1 ist es bereits bekannt, beim Schneiden eines kornealen Flaps zusätzlich einen Gewebestreifen außerhalb des Flaprands wegzuschneiden, um eine Stauchung des Flaps beim Zurückklappen auf das korneale Gewebe zu vermeiden, nachdem unterhalb des Flaps zuvor stromales Gewebe mittels Laserstrahlung abgetragen wurde. Im Querschnitt besitzt der zusätzlich weggeschnittene Gewebestreifen ausweislich der Darstellung in Figur 6 der DE 10 2009 009 382 A1 im wesentlichen konstante Dicke in Richtung von der Korneavorderfläche hin zu tieferen Korneabereichen.

Aufgabe der Erfindung ist es, LASIK-Operationen des humanen Auges, insbesondere solche zur Behebung einer Myopie, bei möglichst geringen Visusbeeinträchtigungen angenehm für den Patienten zu gestalten.

Diese Aufgabe wird durch eine Einrichtung gemäß Anspruch 1 gelöst. Bevorzugte Weiterbildungen sind in den abhängigen Unteransprüchen angegeben.

Eine erfindungsgemäße Einrichtung zur schneidenden Bearbeitung der humanen Kornea mit fokussierter Laserstrahlung umfasst steuerbare Komponenten zur Ortseinstellung des Strahlungsfokus, einen Steuerrechner zur Steuerung dieser Komponenten und ein Steuerprogramm für den Steuerrechner. Das Steuerprogramm enthält Instruktionen, welche dazu ausgelegt sind, bei Ausführung durch den Steuerrechner die Erzeugung von Schnitten in der Kornea nach Maßgabe einer vorgegebenen Schnittfigur zur bewirken, wobei die Schnittfigur ein korneales Bett, einen auf dem Bett liegenden Flap sowie mindestens einen im Bereich des Umfangsrands des Flaps zwischen dem Bett und dem Flap liegenden, längs des Flaprands sich erstreckenden kornealen Gewebestreifen definiert.

Die Erfindung beruht auf der Erkenntnis, dass durch gezielte Kürzung des Flaps das Entstehen von Striae besser vermieden werden kann, so dass anschließende aufwändige Glättungsmaßnahmen an der Kornea entfallen können. Die Kürzung des Flaps ist zweckmäßigerweise so, dass nach der ablativen Behandlung der Flap exakt in das korneale (stromale) Bett hineinpasst und keine oder zumindest nur unwesentliche Falten wirft. Zur Kürzung des Flaps wird am Umfangsrand des Flaps mindestens ein Gewebestreifen freigeschnitten, der nach dem Aufklappen des Flaps entfernt wird. Die Schnittfigur sieht zweckmäßigerweise eine vollständige Separation dieses Gewebestreifens von dem Flap und dem umliegenden kornealen Bett vor. Ja nachdem, ob und in welchem Umfang der Gewebestreifen nach Anbringung der Schnitte noch über schmale Gewebestege zwischen aufeinanderfolgenden Photodisruptionen mit benachbartem Gewebe zusammenhängt, kann es sein, dass beim Aufklappen des Flaps der Gewebestreifen entweder mit dem Flap mitgeht oder in dem Bett liegen bleibt. Für den Operateur ist es jedenfalls gleichermaßen einfach, den Gewebestreifen zu entfernen, sei es, indem er ihn vom Bett abzieht oder vom Flap.

Der Gewebestreifen kann sich im wesentlichen über die gesamte Umfangslänge des Flaprands erstrecken, also im wesentlichen über die gesamte Länge des Rands von einem Ende des Scharniers (Hinge) zum anderen. Alternativ kann sich der Gewebestreifen nur über einen Teil der Umfangslänge des Flaprands erstrecken, wobei es sogar vorstellbar ist, dass die Schnittfigur eine Mehrzahl von mindestens zwei Gewebestreifen definiert, die sich längs unterschiedlicher Umfangsbereiche des Flaprands erstrecken. Anzahl und Umfangslänge der Gewebestreifen hängen vor allem vom Ablationsprofil ab, das häufig nicht rotationssymmetrisch ist, sondern beispielsweise bei Vorhandensein eines Astigmatismus in Umfangsrichtung unsymmetrisch sein kann. Solche Unsymmetrien können sich dann auch in einem veränderlichen Querschnitt des Gewebestreifens in Umfangsrichtung des Flaprands niederschlagen.

Der Gewebestreifen kann vollständig unterhalb der Korneaoberfläche liegen, so dass eine Kürzung des Flaps nur unterhalb der Korneavorderfläche stattfindet. Es ist selbstverständlich gleichermaßen vorstellbar, dass der Gewebestreifen bis zur Korneavorderfläche reicht und dort eine nicht verschwindende, endliche Breite besitzt. In diesem Fall findet eine - geringe - Flapkürzung auch an der Korneavorderfläche statt. Dies kann je nach Stärke des Materialabtrags bei der späteren Ablation erforderlich sein.

In Anbetracht der bei der Behandlung einer Myopie post-ablativ verringerten Bogenlänge der optischen Zone ist es zweckmäßig, wenn der Querschnitt des Gewebestreifens bei Betrachtung in Richtung von der Korneavorderfläche hin zu tieferen Bereichen der Kornea eine zunehmende Breite besitzt. Der Querschnitt des Gewebestreifens kann beispielsweise annähernd keilförmig sein.

Zur Präparation des Flaps und des Gewebestreifens können die Schnitte einen die Flapunterseite definierenden, vollständig in der Tiefe der Kornea liegenden, vorzugsweise parallel zur Korneavorderfläche verlaufenden ersten Schnitt sowie zwei im Abstand voneinander insbesondere winkelig auf den ersten Schnitt stoßende, zwischen sich und dem ersten Schnitt den Gewebestreifen begrenzende zweite Schnitte umfassen, von denen mindestens einer zur Korneavorderfläche herausgeführt ist. Dabei können die beiden zweiten Schnitte unterhalb der Korneavorderfläche aufeinander stoßen. Soll der Gewebestreifen bis zur Korneavorderfläche reichen, können dagegen die beiden zweiten Schnitte unmittelbar auf der Korneavorderfläche aufeinander stoßen oder im Abstand voneinander bis zur Korneavorderfläche herausgeführt sein, ohne einander zu schneiden.

Gemäß einer bevorzugten Ausgestaltung kann der Steuerrechner Zugriff auf Ablationsdaten haben, welche für ein korneales Ablationsprofil repräsentativ sind, wobei der Steuerrechner dazu eingerichtet ist, auf Grundlage der Ablationsdaten die Schnittfigur, insbesondere den Querschnitt des Gewebestreifens in Abhängigkeit vom Umfangsort des Flaprands, zu bestimmen. Indem in solcher Weise die Ablationsdaten der die LASIK-Schnitte setzenden Lasereinrichtung zugänglich gemacht werden, kann der zu entfernende Gewebestreifen nach Form und Größe optimal festgelegt werden. Es versteht sich jedoch, dass der Querschnitt des Gewebestreifens, also seine Form und seine Größe, anstatt auf Grundlage patientenindividueller Ablationsdaten auf der Basis empirischer Daten oder bestimmter theoretischer Modelle festgelegt werden kann.

Ein Verfahren zur Bearbeitung eines humanen Auges umfasst nach einem weiteren Gesichtspunkt die folgenden Schritte:

-   Erzeugen von Schnitten in der Kornea des Auges mittels erster fokussierter Laserstrahlung nach Maßgabe einer vorgegebenen Schnittfigur, wobei die Schnittfigur ein korneales Bett, einen auf dem Bett liegenden Flap sowie mindestens einen im Bereich des Umfangsrands des Flaps zwischen dem Bett und dem Flap liegenden, längs des Flaprands sich erstreckenden kornealen Gewebestreifen definiert,
-   Aufklappen des Flaps,
-   Entfernen des mindestens einen Gewebestreifens,

- Ablatieren des freigelegten Bettgewebes mittels zweiter fokussierter Laserstrahlung nach Maßgabe eines Ablationsprofils,
- Zurückklappen des Flaps.

Das Verfahren kann ferner den Schritt der Bestimmung der Schnittfigur auf Grundlage des Ablationsprofils umfassen. Die Bestimmung der Schnittfigur kann das Ermitteln einer nach der Ablation im Vergleich zu vor der Ablation bestehenden Längendifferenz mindestens einer quer über mindestens einen Teil der Bettoberfläche gemessenen Strecke sowie ein Festlegen des Querschnitts des Gewebestreifens auf Grundlage der ermittelten Längendifferenz umfassen. Die quer über die Bettoberfläche gemessene Strecke ist beispielsweise eine, die durch das Zentrum der ablativ behandelten optischen Zone von einem Zonenrand zum gegenüberliegenden reicht. Die Länge dieser Strecke entspricht insoweit der quer durch das Zentrum gemessenen Bogenlänge der optischen Zone. Soweit ein rotationsunsymmetrischer Materialabtrag im Rahmen der Ablation erfolgen soll, empfiehlt es sich, die Differenz der Bogenlänge (d.h. vor gegenüber nach der Ablation) für eine Vielzahl unterschiedlicher Winkellagen zu ermitteln, beispielsweise unter Nutzung topografischer Daten der Korneavorderfläche oder der Bettoberfläche, um so die Gewebestreifengeometrie individuell anpassen zu können. Dies ermöglicht eine optimale Bestimmung des Querschnittsverlaufs des Gewebestreifens in Umfangsrichtung und damit eine optimale Anpassung des Streifenquerschnitts an die Gegebenheiten des individuellen Patienten.

Die Erfindung wird nachfolgend anhand der beigefügten Zeichnungen weiter erläutert. Es stellen dar:

Fig. 1 in schematischer Blockdarstellung ein Ausführungsbeispiel einer Lasereinrichtung zur Anbringung intrakornealer Schnitte,

Fig. 2 schematisch die Verhältnisse vor und nach der Ablation bei einer LASIK-Behandlung zur Korrektur einer Myopie,

Fig. 3 schematisch einen am Rand keilförmig verkürzten LASIK-Flap und

Fig. 4-6 verschiedene Varianten der Schnittsetzung zur Erzeugung eines verkürzten LASIK-Flaps.

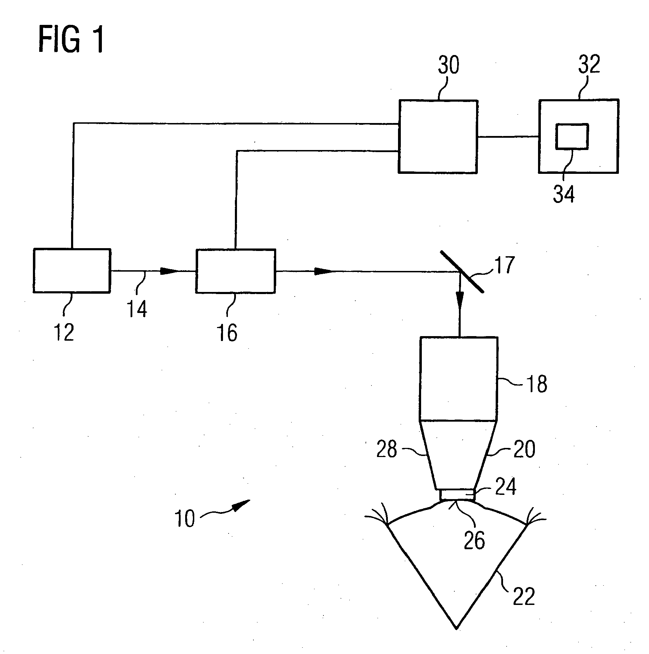

Die in Fig. 1 gezeigte, allgemein mit 10 bezeichnete Lasereinrichtung umfasst eine Laserquelle 12, welche einen Laserstrahl 14 mit Pulsdauern im Femtosekundenbereich erzeugt. Im Strahlengang des Laserstrahls 14 sind eine Reihe von Komponenten angeordnet, unter anderem ein hier schematisch als einheitlicher Funktionsblock angedeutetes Scanmodul 16, ein unbeweglicher Umlenkspiel 17 sowie ein Fokussierobjektiv 18. Das Scanmodul 16 dient zur transversalen und longitudinalen Ortssteuerung des Fokuspunkts des Laserstrahls 14. Transversal bezeichnet hier eine Richtung quer zur Ausbreitungsrichtung des Laserstrahls 14, longitudinal meint entlang der Strahlausbreitungsrichtung. Zur Transversalablenkung des Laserstrahls 14 kann das Scanmodul 16 beispielsweise ein Paar galvanometrisch betätigter Scannerspiegel umfassen, die um zueinander senkrechte Achsen kippbar sind. Alternativ ist beispielsweise eine Transversalablenkung mittels eines elektrooptischen Kristalls denkbar.

Für die Longitudinalsteuerung der Fokusposition kann das Scanmodul 16 beispielsweise eine longitudinal verstellbare oder brechkraftvariable Linse oder einen deformierbaren Spiegel enthalten, mit der bzw. mit dem sich die Divergenz des Laserstrahls 14 und folglich die Longitudinalposition des Strahlfokus beeinflussen lässt.

Es versteht sich, dass die für die transversale Ortseinstellung und die longitudinale Ortseinstellung des Fokus dienenden Komponenten des Scanmoduls 16 entlang des Strahlengangs des Laserstrahls 14 verteilt angeordnet und insbesondere in unterschiedlichen Baueinheiten untergebracht sein können. Beispielsweise kann die Funktion der longitudinalen Fokussteuerung von einer in einer Strahlaufweitungsoptik (Beam Expander, z.B. Galilei-Teleskop) angeordneten Linse ausgefüllt werden, während die für die transversale Fokussteuerung dienenden Komponenten in einer gesonderten Baueinheit zwischen der Strahlaufweitungsoptik und dem Fokussierobjektiv 18 untergebracht sein können. Die Darstellung des Scanmoduls 16 als einheitlicher Funktionsblock in Fig. 1 dient lediglich der besseren Übersichtlichkeit.

Das Fokussierobjektiv 18 ist bevorzugt ein F-Theta-Objektiv und ist auf seiner Strahlaustrittsseite mit einem Patientenadapter 20 vorzugsweise lösbar gekoppelt, welcher eine Anlageschnittstelle für die Kornea eines zu behandelnden Auges 22 bildet. Der Patientenadapter 20 weist hierzu ein für die Laserstrahlung transparentes Kontaktelement 24 auf, welches auf seiner augenzugewandten Unterseite eine Anlagefläche (Kontaktfläche) 26 für die Kornea aufweist. Die Anlagefläche 26 ist im gezeigten Beispielfall als Planfläche ausgeführt und dient zur Einebnung der Kornea, indem das Kontaktelement 24 mit entsprechendem Druck gegen das Auge 22 gedrückt wird oder die Kornea durch Unterdruck an die Kontaktfläche 26 angesaugt wird. Das Kontaktelement 24 (bei planparalleler Ausführung üblicherweise als Applanationsplatte bezeichnet) ist am schmalen Ende eines Abstandskegels 28 angebracht. Die Verbindung zwischen dem Kontaktelement 24 und dem Abstandskegel 28 kann unlösbar sein, beispielsweise durch Verklebung. Alternativ kann sie lösbar sein, etwa durch eine Verschraubung. Der Abstandskegel 28 besitzt in nicht näher dargestellter Weise an seinem breiten Ende geeignete Koppelformationen zur longitudinal und transversal positionsstabilen Ankopplung an das Fokussierobjektiv 18.

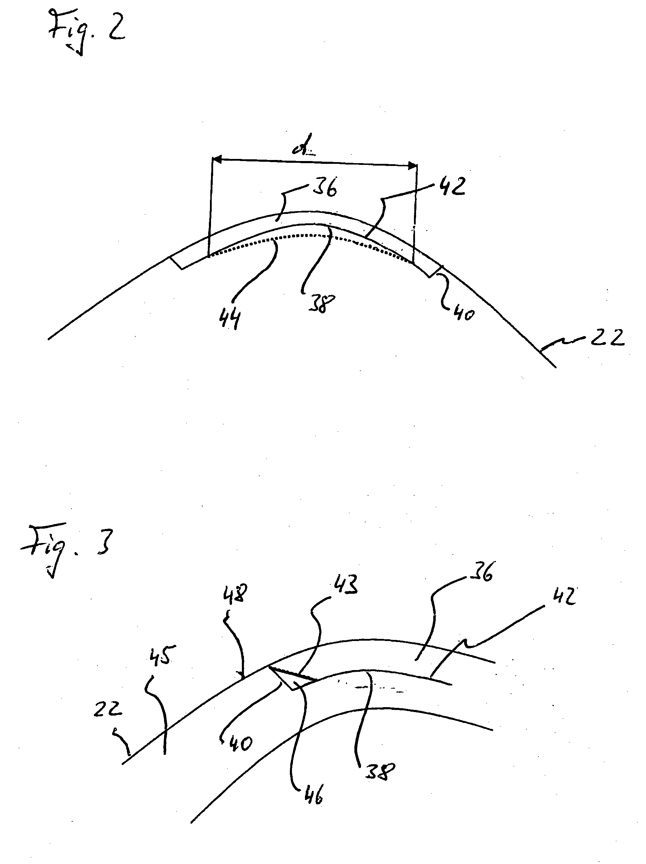

Die Laserquelle 12 und das Scanmodul 16 werden von einem Steuerrechner 30 gesteuert, der nach Maßgabe eines in einem Speicher 32 gespeicherten Steuerprogramms 34 arbeitet. Das Steuerprogramm 34 enthält Instruktionen (Programmcode), welche bei Ausführung durch den Steuerrechner 30 eine solche Ortssteuerung des Strahlfokus des Laserstrahls 14 bewirken, dass in der Kornea des an dem Kontaktelement 24 anliegenden Auges 22 ein LASIK-Flap entsteht. Bevor auf Einzelheiten dieses Flaps eingegangen wird, sei kurz noch auf Fig. 2 verwiesen, wo für das Auge 22 schematisch ein herkömmlicher kornealer Flap 36 gezeigt ist, der durch einen Bettschnitt 38 und einen Randschnitt 40 vom übrigen Korneagewebe getrennt ist und satt in dem durch die Schnitte 38, 40 begrenzten stromalen Bett liegt. Dieses Bett ist hier mit 42 bezeichnet.

In Fig. 2 sei der Fall angenommen, dass das Bett 42 in einer optischen Zone mit dem Durchmesser d ablatierend mit geeigneter UV-Laserstrahlung behandelt wird. Es versteht sich, dass der Flap 36 hierzu zuvor zur Seite geklappt werden muss, um die zu bearbeitende optische Zone zu exponieren. Die Ablation soll zur Korrektur einer Myopie dienen, d.h. der Materialabtrag ist im Zentrum der optischen Zone am größten und nimmt zu den Rändern der optischen Zone hin ab. Post-ablativ stellt sich daher eine Oberfläche des Betts 42 ein, wie sie in beispielhafter Form punktiert bei 44 angedeutet ist. Es ist ohne weiteres ersichtlich, dass die quer durch das Zentrum der optischen Zone an der Oberfläche des Betts von Rand zu Rand gemessene Strecke post-ablativ kürzer ist als prä-ablativ. Dies gilt für die Strecke Rand zu Rand der ablatierten optischen Zone genauso wie für die Strecke Rand zu Rand des Betts insgesamt. Soweit der Materialabtrag rotationssymmetrisch ist, ist die Verkürzung der Bogenlänge der Bettoberfläche in allen Meridianrichtungen zumindest näherungsweise gleich. Bei einem komplexeren Ablationsprofil, das in unterschiedlichen Meridianrichtungen unterschiedliche Stärken des Abtrags verlangt, kann die Bogenlängendifferenz der Bettoberfläche zwischen prä- ablativem und post-ablativem Zustand entsprechend variieren.

Ungeachtet dessen hat die Verkürzung der Bogenlänge der Bettoberfläche zur Folge, dass der Flap 36 nach der Ablation sich nicht satt in das - nunmehr abgesenkte-Bett 42 einfügen kann: Weil die Flap-Unterseite im Bereich der optischen Zone eine größere Bogenlänge hat als die ablatierte Bettoberfläche, legt sich der Flap 36 beim Zurückklappen nicht vollflächig an die Bettoberfläche an. Stattdessen wirft er kleinere Falten (Mikrostriae). Ohne anschließende Zusatzmaßnahmen verbleiben diese Mikrostriae und beeinträchtigen die Sehschärfe zum Teil beträchtlich.

Es versteht sich, dass die im Zusammenhang mit Fig. 2 angestellten Betrachtungen sich auf den nicht applanierten Zustand des Auges beziehen, also auf einen Zustand, in dem das Auge 22 nicht mehr an der Kontaktplatte 24 der Lasereinrichtung der Fig. 1 anliegt.

Um eine verbesserte post-ablative Anschmiegung des LASIK-Flaps an das stromale Bett zu erreichen, sieht die durch das Steuerprogramm 34 repräsentierte Schnittfigur eine randseitige Verkürzung des Flaps vor, indem dort ein näherungsweise keilförmiger Gewebestreifen vom Flaprand abgetrennt wird. Diesbezüglich wird nun auf Fig. 3 verwiesen. Trotzdem der dort gezeigte Flap ein erfindungsgemäß gekürzter Flap ist, werden aus Gründen der Übersichtlichkeit dennoch in Fig. 3 und den nachfolgenden Figuren gleiche Bezugszeichen wie in Fig. 2 verwendet.

Die Schnittfigur der Fig. 3 umfasst neben dem Bettschnitt 38 und dem Randschnitt 40 noch einen Keilschnitt 43, welcher radial (bezogen auf ein nicht näher dargestelltes, gedachtes Zentrum der mit 45 bezeichneten Kornea des Auges 22) zumindest weitestgehend innerhalb des Randschnitts 40 in Umfangsrichtung des Flaprands verläuft und zusammen mit dem Randschnitt 40 und dem Bettschnitt 38 einen im Querschnitt näherungsweise keilförmigen Gewebestreifen 46 begrenzt, der nach dem Hochheben und Wegklappen des Flaps 36 entnommen werden kann. Der Bettschnitt 38 verläuft im gezeigten Beispielfall in im wesentlichen gleichmäßiger Tiefe der Kornea 45 parallel zu der mit 48 bezeichneten Korneavorderfläche. Der Randschnitt 40 sowie der Keilschnitt 43 verlaufen winkelig zu dem Bettschnitt 38 in Richtung zur Korneavorderfläche 48. Der radiale Abstand zwischen dem Randschnitt 40 und dem Keilschnitt 43 ist im Bereich des Bettschnitts 38 am größten; bei Voranschreiten in Richtung zur Korneavorderfläche 48 nähern sich der Randschnitt 40 und der Keilschnitt 43 einander an.

Die Größe des von dem Streifen 46 gebildeten Gewebekeils hängt von der postablativen Bogenlängenverringerung der Bettoberfläche in der betreffenden Meridianrichtung ab. Darüber hinaus hängt sie davon ab, ob diese Bogenlängenreduzierung durch einen einzigen Gewebekeil oder durch zwei in diametral gegenüberliegenden Randbereichen des Flaps liegende Gewebekeile ausgeglichen werden kann. In denjenigen Randbereichen des Flaps, die dem Hinge gegenüberliegen, muss die gesamte Bogenlängendifferenz in der betreffenden Richtung durch einen einzigen Gewebekeil kompensiert werden. In den übrigen Meridianrichtungen kann die Bogenlängendifferenz durch zwei Gewebekeile an einander gegenüberliegenden Randstellen des Flaps kompensiert werden. Dementsprechend kann die Größe und Form (oder allgemein: der Querschnitt) des Gewebestreifens 46 bei Fortschreiten in Umfangsrichtung des Flaprands variieren. Insbesondere kann der Gewebestreifen 46 in denjenigen Randbereichen, die dem Hinge gegenüberliegen, einen größeren Querschnitt besitzen als in den übrigen Umfangsbereichen.

Je nach Ablationsprofil kann sich der Gewebestreifen 46 über den gesamten Umfang des Flaprands erstrecken. Es ist auch vorstellbar, dass sich der Gewebestreifen 46 nur längs eines Teilstücks des Flaprands erstreckt. Es ist sogar vorstellbar, längs des Flaprands mehrere, in Umfangsrichtung im Abstand voneinander liegende Gewebestreifen 46 zu erzeugen.

Wenngleich in Fig. 3 und den nachfolgenden Figuren der Randschnitt 40 und der Keilschnitt 43 jeweils als im Querschnitt geradlinige Schnitte dargestellt sind, so versteht es sich, dass dies keineswegs zwingend ist. Insbesondere für den Keilschnitt 43 kann ohne weiteres auch ein nicht gerader Schnittverlauf gewählt werden.

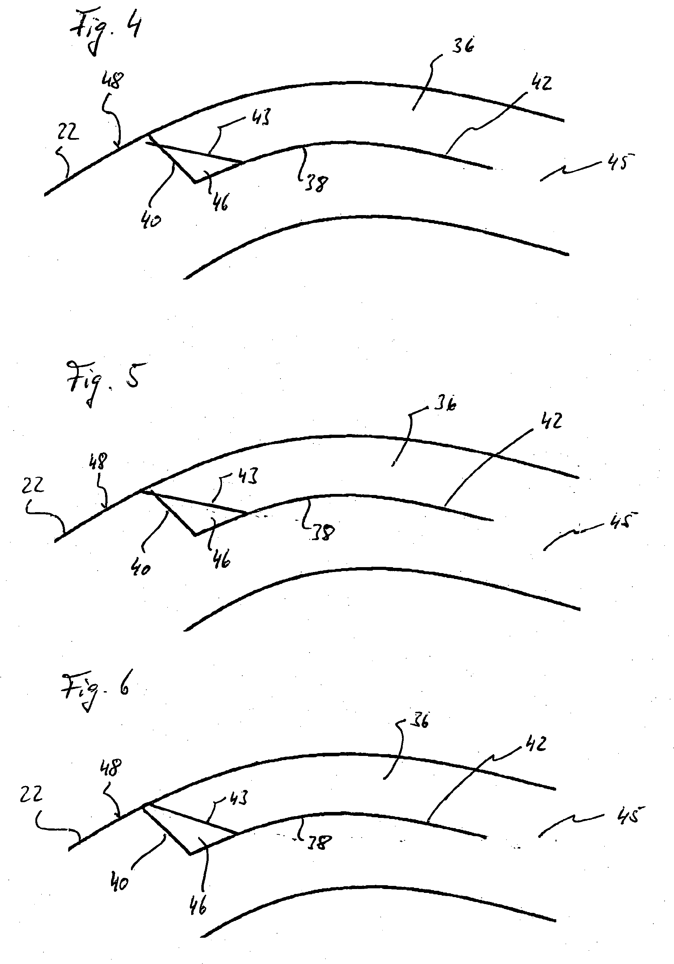

Im Beispielfall der Fig. 3 treffen der Randschnitt 40 und der Keilschnitt 43 im wesentlichen unmittelbar an der Korneavorderfläche 48 aufeinander. Die Figuren 4 bis 6 zeigen verschiedene Abwandlungen für den relativen Verlauf des Randschnitts 40 und des Keilschnitts 43.

In Fig. 4 schneiden sich der Randschnitt 40 sowie der Keilschnitt 43 unterhalb der Korneavorderfläche 48, wobei der Keilschnitt 43 im Abstand von der Korneavorderfläche 48 endet und der Randschnitt 40 bis zur Korneavorderfläche 48 durchgezogen ist.

Bei der Variante der Fig. 5 schneiden sich der Randschnitt 40 und der Keilschnitt 43 gleichermaßen unterhalb der Korneavorderfläche 48, wobei in diesem Fall freilich der Randschnitt 40 im Abstand vor der Korneavorderfläche 48 endet und stattdessen der Keilschnitt 43 bis zur Korneavorderfläche 48 durchgezogen ist.

Die Abwandlung gemäß Fig. 6 zeigt einen Fall, bei dem sowohl der Randschnitt 40 als auch der Keilschnitt 43 bis zur Korneavorderfläche 48 durchgehen und im Abstand voneinander auf die Korneavorderfläche 48 stoßen, ohne freilich einander zu schneiden.

Indem der Flap in vorstehend erläuterter Weise an seinem Rand vorzugsweise keilförmig gekürzt wird, ist es möglich, ihn so zu modifizieren, dass er exakt in das post-ablative stromale Bett hineingelegt werden kann. Wie erläutert, kann die Kürzung am gesamten Flap - mit Ausnahme derjenigen Bereiche, wo sich der Hinge befindet - vorgenommen werden. Die Berechnung des Querschnitts des Gewebestreifens 46, d.h. allgemein die Berechnung der kornealen Schnittfigur, kann unter Berücksichtigung der Größe der ablativ behandelten optischen Zone, der Brechkräfte der Kornea vor und nach der Ablation sowie der Asphärizitäten der Korneavorderfläche durchgeführt werden. Eine mögliche Berechnungsgrundlage geben die nachstehend wiedergegebenen mathematischen Formeln.

$$P = \frac{n-1}{R} \rightarrow R_1 = \frac{n-1}{P_{vorOP}}; R_2 = \frac{n-1}{P_{nachOP}}; R_2 > R_1 \qquad [1]$$

$$s = \frac{1}{1+Q_1}\left[R_1^2 - y^2(1+Q_1)\right]^{1/2} - \frac{1}{1+Q_2}\left[R_2^2 - y^2(1+Q_2)\right]^{1/2}$$

$$+ \frac{1}{1+Q_2}\left[R_2^2 - \frac{OZ^2}{4}(1+Q_2)\right]^{1/2} - \frac{1}{1+Q_1}\left[R_1^2 - \frac{OZ^2}{4}(1+Q_1)\right]^{1/2} \qquad [2]$$

$$b_1 = 2R_1 * \arcsin\left(\frac{OZ}{2R_1}\right)$$

$$b_2 = 2R_2 \arcsin\sqrt{\left(1 - \left(\frac{s(y=0) - R_1 + R_2 + \sqrt{R_1^2 - \frac{OZ^2}{4}}}{R_2}\right)^2\right)}$$

$$\Delta b = b_1 - b_2$$

$P_{vorOP}$: Brechkraft der Kornea vor der Operation (z.B. $P_{vorOP}$ = 43 dpt)

$P_{nachOP}$: gewünschte Brechkraft der Kornea nach der Operation

$R_1$: Krümmungsradius der optischen Zone vor der Ablation

$R_2$: Krümmungsradius der optischen Zone nach der Ablation

n: Brechzahl der Kornea (n≈1,377)

$b_1$: Bogenlänge der optischen Zone vor der Ablation

$b_2$: Bogenlänge der optischen Zone nach der Ablation

OZ: Durchmesser der optischen Zone

$Q_1$: Asphärizität der Korneavorderfläche vor der Ablation ($-1<Q_1<1$)

$Q_2$: Asphärizität der Korneavorderfläche nach der Ablation ($-1<Q_2<1$)

s: Tiefe der Ablation

y: radiale Laufvariable (y=0 an der Stelle maximaler Ablation, d. h. Ablationszentrum)

Mit Hilfe der obigen mathematischen Grundlagen kann für einen rein zentralen (rotationssymmetrischen) Materialabtrag unter Berücksichtigung der Asphärizitäten der Korneavorderfläche die Bogenlängendifferenz $\Delta b$ (post-ablativ gegenüber prä-ablativ) der optischen Zone berechnet werden. Mit Kenntnis der Bogenlängendifferenz ist es ohne weiteres möglich, den Querschnitt des zu entfernenden Gewebestreifens 46 zu berechnen. Dabei ist, wie erläutert, zu berücksichtigen, dass diametral zum Hinge die Kürzung durch einen einzigen Gewebekeil erfolgen muss, während an den übrigen Seiten des Flaps die Kürzung auf zwei Gewebekeile aufgeteilt werden kann.

Rein beispielhaft wurde unter der Annahme von Asphärizitätswerten $Q_1=Q_2=-0,3$ und einer Größe (Durchmesser) der optischen Zone von 6,5 mm das folgende Zahlentableau rechnerisch ermittelt. Dies gibt für unterschiedliche zu korrigierende Werte der Kurzsichtigkeit die resultierende Bogenlängendifferenz der optischen Zone an. Diese Zahlenwerte wurden unter Verwendung der vorstehend wiedergegebenen mathematischen Grundlagen berechnet.

| zu korrigierende Werte [dpt] | Differenz der Bogenlänge [$\mu m$] |
| --- | --- |
| 1 | 5 |
| 2 | 10 |
| 3 | 14 |
| 4 | 19 |
| 5 | 23 |
| 6 | 27 |
| 7 | 30 |
| 8 | 34 |

Vorzugsweise hat der Steuerrechner 30 der Lasereinrichtung gemäß Fig. 1 Zugriff auf geeignete Ablationsdaten, welche für den zu realisierenden Materialabtrag repräsentativ sind. Die Ablationsdaten können beispielsweise in dem Speicher 32 abgespeichert sein. Dabei ist es vorstellbar, dass der Speicher 32 auch für einen Steuerrechner einer nicht näher dargestellten gesonderten Lasereinrichtung für die spätere ablatierende Behandlung des Auges zugänglich ist. Von dem Steuerrechner dieser Ablations-Lasereinrichtung können die Ablationsdaten in den Speicher 32 geschrieben werden, wobei der Steuerrechner 30 der in Fig. 1 gezeigten Schneid-Lasereinrichtung auf Grundlage der Ablationsdaten die für den jeweiligen Patienten geeignete Schnittfigur berechnet.

## Patentansprüche

1. Einrichtung zur schneidenden Bearbeitung der humanen Kornea (45) mit fokussierter Laserstrahlung, mit steuerbaren Komponenten (16) zur Ortseinstellung des Strahlungsfokus, einem Steuerrechner (30) zur Steuerung dieser Komponenten und einem Steuerprogramm (34) für den Steuerrechner, wobei das Steuerprogramm Instruktionen enthält, welche dazu ausgelegt sind, bei Ausführung durch den Steuerrechner die Erzeugung von Schnitten (38, 40, 43) in der Kornea nach Maßgabe einer vorgegebenen Schnittfigur zu bewirken, wobei die Schnittfigur ein korneales Bett (42), einen auf dem Bett liegenden Flap (36) sowie mindestens einen im Bereich des Umfangsrands des Flaps zwischen dem Bett und dem Flap liegenden, längs des Flaprands sich erstreckenden kornealen Gewebestreifen (46) definiert,
dadurch gekennzeichnet, dass der Querschnitt des Gewebestreifens (46) bei Betrachtung in Richtung von der Korneavorderfläche (48) hin zu tieferen Bereichen der Kornea (45) zunehmende Breite besitzt.

**2.** Einrichtung nach Anspruch 1, wobei sich der Gewebestreifen (46) im wesentlichen über die gesamte Umfangslänge des Flaprands erstreckt.

**3.** Einrichtung nach Anspruch 1, wobei sich der Gewebestreifen (46) nur über einen Teil der Umfangslänge des Flaprands erstreckt.

**4.** Einrichtung nach Anspruch 3, wobei die Schnittfigur eine Mehrzahl von mindestens zwei Gewebestreifen definiert, die sich längs unterschiedlicher Umfangsbereiche des Flaprands erstrecken.

**5.** Einrichtung nach einem der vorhergehenden Ansprüche, wobei der Gewebestreifen (46) vollständig unterhalb der Korneavorderfläche (48) liegt.

**6.** Einrichtung nach einem der vorhergehenden Ansprüche, wobei der Gewebestreifen (46) in Umfangsrichtung des Flaprands veränderlichen Querschnitt besitzt.

**7.** Einrichtung nach einem der vorhergehenden Ansprüche, wobei der Querschnitt des Gewebestreifens (46) näherungsweise keilförmig ist.

**8.** Einrichtung nach einem der vorhergehenden Ansprüche, wobei die Schnitte (38, 40, 43) einen die Flapunterseite definierenden, vollständig in der Tiefe der Kornea (45) liegenden, vorzugsweise parallel zur Korneavorderfläche (48) verlaufenden ersten Schnitt (38) sowie zwei im Abstand voneinander insbesondere winkelig auf den ersten Schnitt stoßende, zwischen sich und dem ersten Schnitt den Gewebestreifen (46) begrenzende zweite Schnitte (40, 43) umfassen, von denen mindestens einer zur Korneavorderfläche herausgeführt ist.

**9.** Einrichtung nach Anspruch 8, wobei die beiden zweiten Schnitte (40, 43) unterhalb der Korneavorderfläche (48) aufeinander stoßen.

**10.** Einrichtung nach einem der vorhergehenden Ansprüche, wobei der Steuerrechner (30) Zugriff auf Ablationsdaten hat, welche für ein korneales Ablationsprofil repräsentativ sind, wobei der Steuerrechner dazu eingerichtet ist, auf Grundlage der Ablationsdaten die Schnittfigur, insbesondere den Querschnitt des Gewebestreifens (46) in Abhängigkeit vom Umfangsort des Flaprands, zu bestimmen.

## Claims

**1.** A device for machining the human cornea (45) with focused laser radiation, comprising controllable components (16) for setting the location of the radiation focus, a control computer (30) for controlling these components and a control program (34) for the control computer, wherein the control program contains instructions which, upon execution by the control computer, are configured to bring about the generation of incisions (38, 40, 43) in the cornea in accordance with a predetermined incision figure, wherein the incision figure defines a corneal bed (42), a flap (36) situated on the bed as well as at least one tissue strip (46) extending in the region of a peripheral edge of the flap between the bed and the flap and along the edge of flap,
**characterised in that** the cross-section of the tissue strip (46) has an increasing width when viewed in the direction from the anterior surface (48) of the cornea towards deeper regions of the cornea (45).

**2.** The device according to Claim 1, wherein the tissue strip (46) extends essentially over the entire peripheral length of the edge of the flap.

**3.** The device according to Claim 1, wherein the tissue strip (46) extends only over a portion of the peripheral length of the edge of the flap.

**4.** The device according to Claim 3, wherein the incision figure defines a plurality of at least two tissue strips which extend along different peripheral regions of the edge of the flap.

**5.** The device according to one of the previous claims, wherein the tissue strip (46) is situated completely beneath the anterior surface (48) of the cornea.

**6.** The device according to one of the previous claims, wherein the tissue strip (46) has a variable cross-section in the peripheral direction of the edge of the flap.

7. The device according to one of the previous claims, wherein the cross-section of the tissue strip (46) is approximately wedge-shaped.

8. The device according to one of the previous claims, wherein the incisions (38, 40, 43) comprise a first incision (38) which defines the underside of the flap and is situated totally deep within the cornea (45), preferably extending parallel to the anterior surface (48) of the cornea, as well as two second incisions (40, 43) which are spaced from one another and, in particular, meet the first incision under an angle, defining the tissue strip (46) between them and the first incision, of which at least one is extended outwards to the anterior surface of the cornea.

9. The device according to Claim 8, wherein the two second incisions (40, 43) meet beneath the anterior surface (48) of the cornea.

10. The device according to one of the previous claims, wherein the control computer (30) may access ablation data which are representative for a corneal ablation profile, wherein the control computer is configured to determine the incision figure, in particular the cross-section of the tissue strip (46), depending on the peripheral location of the edge of the flap on the basis of the ablation data of the incision figure.

## Revendications

1. Dispositif de traitement par incision de la cornée humaine (45) à l'aide d'un faisceau laser focalisé, comprenant des éléments contrôlables (16) destinés au réglage spatial du foyer du faisceau, un calculateur de commande (30) pour commander ces éléments et un programme de commande (34) pour le calculateur de commande, ce programme de commande contenant des instructions qui sont conçues pour réaliser dans la cornée, suivant un motif de découpe prédéfini, des incisions (38, 40, 43) lorsque sont exécutées ces instructions par le calculateur de commande, ledit motif de découpe définissant un lit cornéen (42), un volet cornéen (36) reposant sur ce lit et au moins une bande (46) de tissu cornéen s'étendant le long du bord du volet cornéen et située dans la zone du bord périphérique du volet cornéen, entre le lit et le volet cornéen,
**caractérisé en ce que** la section de la bande (46) de tissu cornéen, vue en partant de la face avant (48) de la cornée vers les zones plus profondes de la cornée (45), possède une largeur croissante.

2. Dispositif selon la revendication 1, la bande de tissu (46) s'étendant sensiblement sur toute la longueur circonférentielle du bord du volet.

3. Dispositif selon la revendication 1, la bande de tissu (46) ne s'étendant que sur une partie de la longueur circonférentielle du bord du volet.

4. Dispositif selon la revendication 3, le motif de découpe définissant une pluralité de bandes de tissus - leur nombre étant au moins égal à deux - qui s'étendent le long de différentes zones circonférentielles du bord du volet.

5. Dispositif selon l'une des revendications précédentes, la bande de tissu (46) se trouvant entièrement sous la face avant (48) de la cornée.

6. Dispositif selon l'une des revendications précédentes, la bande de tissu (46) possédant une section variable dans la direction circonférentielle du bord du volet.

7. Dispositif selon l'une des revendications précédentes, la section de la bande de tissu (46) étant approximativement cunéiforme.

8. Dispositif selon l'une des revendications précédentes, les incisions (38, 40, 43) comprenant une première incision (38) définissant la face inférieure du volet, située entièrement dans la profondeur de la cornée (45) et s'étendant de préférence parallèlement à la face avant (48) de la cornée, et deux deuxièmes incisions (40, 43) espacées l'une de l'autre, angulairement jointives avec la première incision et délimitant la bande de tissu (46) entre elles et la première incision, dont au moins une est conduite vers la face avant de la cornée.

9. Dispositif selon la revendication 8, les deux deuxièmes incisions (40, 43) se rejoignant sous la face avant (48) de la cornée.

**10.** Dispositif selon l'une des revendications précédentes, le calculateur de commande (30) ayant accès aux données de l'ablation, lesquelles sont représentatives d'un profil ablatif cornéen, et le calculateur de commande étant conçu pour déterminer, à partir des données de l'ablation, le motif de découpe, plus particulièrement la section de la bande de tissu (46), en fonction de la position périphérique du bord du volet.

FIG 1

Fig. 2

d

36    42

44    38    40    22

Fig. 3

48    43    36    42

45

22    40    38

46

Fig. 4

Fig. 5

Fig. 6

**IN DER BESCHREIBUNG AUFGEFÜHRTE DOKUMENTE**

**In der Beschreibung aufgeführte Patentdokumente**

- DE 102009009382 A1